(19) **Europäisches Patentamt**

**European Patent Office**

**Office européen des brevets**

(11) **EP 0 679 373 B1**

(12) **EUROPEAN PATENT SPECIFICATION**

(45) Date of publication and mention
of the grant of the patent:
**13.01.1999 Bulletin 1999/02**

(51) Int. Cl.$^6$: **A61F 2/06**, A61L 31/00

(21) Application number: **95302858.6**

(22) Date of filing: **27.04.1995**

(54) **Intravascular prosthesis with anti-thrombogenic coating**

Intravaskuläre Prothese mit antithrombogenischer Beschichtung

Prothèse intravasculaire avec revêtement antitrombogène

(84) Designated Contracting States:
**BE CH DE FR GB LI NL**

(30) Priority: **28.04.1994 US 234547**

(43) Date of publication of application:
**02.11.1995 Bulletin 1995/44**

(73) Proprietor:
**ADVANCED CARDIOVASCULAR SYSTEMS, INC.
Santa Clara California 95052 (US)**

(72) Inventors:
• **Dixon, Richard
San Jose, California 95132 (US)**
• **Khosravi, Farhad
312 Newton, Massachusetts 02159 (US)**

• **Lam, Sharon S.
San Jose, California 95129 (US)**
• **Toyloy, Sara
Fremont, California 94538 (US)**

(74) Representative:
**Mayes, Stuart David et al
BOULT WADE TENNANT,
27 Furnival Street
London EC4A 1PQ (GB)**

(56) References cited:
**EP-A- 0 540 290        EP-A- 0 565 251
GB-A- 2 281 865        US-A- 3 843 974
US-A- 5 067 491**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Xerox (UK) Business Services
2.16.7/3.6

## Description

### BACKGROUND OF THE INVENTION

#### Field of the Invention

This invention generally relates to expandable intravascular prosthesis devices, also known as stents, that are implanted in a patient's body lumen, such as a blood vessel, to maintain the patency of the lumen. More particularly, the invention concerns an intravascular prosthesis, such as a stent, coated with a para-xylylene polymer to inhibit development of thrombosis in a blood vessel in which the stent is implanted.

#### Description of Related Art

Stents are generally cylindrically shaped devices which function to hold open and sometimes expand a segment of a blood vessel or other anatomical lumen. They are particularly suitable for supporting and preventing a torn or injured arterial lining from occluding a fluid passageway. Intravascular stents are increasingly useful for treatment of coronary artery stenoses, and for prevention of restenosis or closure after balloon angioplasty.

One such stent is described in EP-A-0,540,290 and comprises a plurality of radially expandable cylindrical elements generally aligned on a common axis and interconnected by one or more interconnective elements. The individual radially expandable cylindrical elements consist of ribbon-like material disposed in an undulating pattern.

This document is used as basis for the preamble of independent claims 1 and 14.

The success of a stent can be assessed by evaluating a number of factors, such as thrombosis; neointimal hyperplasia, primarily due to smooth muscle cell migration and proliferation following implantation of the stent; injury to the artery wall; overall loss of luminal patency; stent diameter in vivo; and leukocyte adhesion to the luminal lining of stented arteries. However, the chief areas of concern are early subacute thrombosis, and eventual restenosis of the blood vessel due to intimal hyperplasia.

It is well known that various types of foreign materials can lead to thrombosis and inflammation. Materials that support endothelial cell growth are generally biocompatible. The para-xylylene polymers, generically known as parylenes, have been used as a coating and as a material for anchoring experimental fabrics used as linings for circulatory assist devices. The terms "Parylene N," "Parylene C," and "Parylene D" are trade names of the Union Carbide Corporation, for specific polymer coatings made from Union Carbide Corporation dimers. While Parylene N has been found to cause unacceptable tissue reactions when used as an electrically-insulating coating for implantable electrodes and circuits, Parylene C is well known for its biocompatability when used as a polymer coating for such implantable electrodes and circuits.

While in vitro tissue culture studies have shown that human cell types readily proliferate on Parylene C-coated surfaces to produce thin layers of normal tissue, other studies have shown that while patches of microfabric coated with Parylene C provided a good support for endothelial cells, such Parylene C-coated material can be thrombogenic, and can become occlusive, necrotic and cause embolisms.

Recent evaluations of currently available stent designs have shown high rates of early subacute thrombosis and later restenosis due to neointimal hyperplasia. While aggressive antithrombotic regimens, including administration of anticlotting drugs such as aspirin and heparin, can limit subacute thrombosis, such regimens can also result in high rates of bleeding and vascular complications. It would therefore be desirable to provide a coated stent that would limit thrombogenicity during a period of re-endothelialization following implantation of the stent, to reduce or obviate the need for administration of high-risk anticoagulants. It also would be desirable to provide an intravascular stent conducive to re-endoetheliazation, and which limits restenosis due to problems of thrombosis and neointimal hyperplasia.

### SUMMARY OF THE INVENTION

According to the present invention there is provided a longitudinally flexible stent for implanting in a body lumen and expandable from a contracted condition to an expanded condition, comprising: a plurality of adjacent cylindrical elements which are independently expandable in a radial direction and arranged in alignment along a longitudinal stent axis, said cylindrical elements being formed in a serpentine wave pattern transverse to the longitudinal axis and containing a plurality of alternating peaks and valleys; and at least one interconnecting member extending between adjacent cylindrical elements and connecting them to one another, characterised in that said serpentine wave pattern is irregular and said stent further comprises an antithrombogenic coating covering said plurality of cylindrical elements and said at least one interconnecting member, said coating comprising a parylene polymer selected from the group consisting of poly-(chloro-para-xylylene), poly-(dichloro-para-xylylene), and poly (para-xylylene). The stent is expandable radially, is relatively flexible along its longitudinal axis to facilitate delivery through tortuous body lumens, and is stable radially in an expanded condition when implanted in a body lumen such as an artery or other vessel, to maintain patency. The cylindrical elements are interconnected to insure that longitudinal contraction of the stent during expansion is minimized.

In a preferred embodiment, the irregular serpentine pattern contains varying degrees of curvature in regions of the peaks and valleys, and is adapted so that radial expansion of the cylindrical elements is generally uniform about the circumferences of each, during the expansion of the stent from its contracted to its expanded condition.

In a presently preferred embodiment, the stent is conveniently and easily formed by coating a stainless steel hypotube with a material resistant to chemical etching, before removing portions of the coating to expose portions of underlying tubing which are to be removed to develop the desired stent structure. The exposed portions of the tubing are removed by chemically etching the tubing exterior, leaving the coated portion of the tubing material in the desired pattern of the stent structure. The etching process develops smooth openings in the tubing wall without burrs or other artifacts which can be characteristic of mechanical or laser machining processes in the small-sized products contemplated. Moreover, a computer-controlled laser patterning process to remove the chemical resistive coating makes photolithography technology adaptable to the manufacture of these small products. A plurality of stents can be formed from one length of hypotube by repeating the stent pattern and providing small webs or tabs to interconnect the stents. After the etching process, the stents can be separated by severing the small webs or tabs which connect them. The stents are then treated by applying a coating of parylene by vapor deposition.

These and other aspects and advantages of the invention will become apparent from the following detailed description, and the accompanying drawings, which illustrate by way of example the features of the invention.

## BRIEF DESCRIPTION OF THE DRAWINGS

FIG. 1 is an elevational view, partially in section, depicting a stent embodying features of the invention which is mounted on a delivery catheter and disposed within a damaged vessel.

FIG. 2 is an elevational view, partially in section, similar to that shown in FIG. 1, wherein the stent is expanded within a damaged vessel, pressing the damaged lining against the vessel wall.

FIG. 3 is an elevational view, partially in section, showing the expanded stent within the vessel after withdrawal of the delivery catheter.

FIG. 4 is an enlarged partial view of the stent of FIG. 5 depicting the irregular serpentine pattern along the peaks and valleys that form the cylindrical elements.

FIG. 5 is a plan view of a flattened section of an embodiment which illustrates the irregular serpentine pattern of the stent.

FIG. 6 is a schematic representation of equipment for selectively removing coating applied to tubing in the manufacturing of the stents embodying the invention.

FIG. 7 is a diagram of the chemical equation involved in the vapor deposition of Parylene C on the stent.

FIG. 8 is a schematic diagram of the process for vapor deposition of Parylene C on the stent.

## DETAILED DESCRIPTION OF THE INVENTION

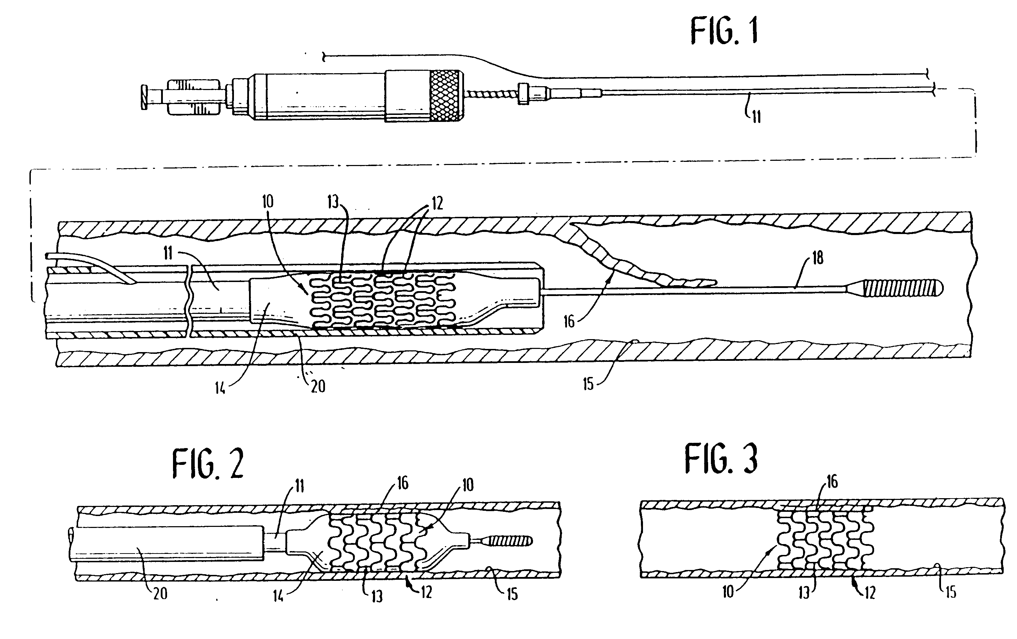

As is illustrated in the drawings, there is provided a stent 10, which is shown mounted onto a delivery catheter 11 in Fig. 1. The stent generally comprises a plurality of radially expandable cylindrical elements 12 disposed generally coaxially and interconnected by members 13 disposed between adjacent cylindrical elements. The delivery catheter 11 has an expandable portion or balloon 14 for expanding of the stent 10 within an artery 15 or other vessel. The artery 15, as shown in Fig. 1, has a separated lining 16 which has occluded a portion of the arterial passageway.

The delivery catheter 11 onto which the stent 10 is mounted essentially can be the same as a conventional balloon dilatation catheter used for angioplasty procedures. The balloon 14 can be formed of suitable materials such as polyethylene, polyethylene terephthalate, polyvinyl chloride, nylon and ionomers such as the ionomer resin sold under the trademark "SURLYN", manufactured by the Polymer Products Division of the DuPont de Nemours Company. Other similar polymers also may be suitable. In order for the stent 10 to remain in place on the balloon 14 during delivery to the site of the damage within the artery 15, the stent 10 is compressed onto the balloon. A retractable protective delivery sleeve 20 may be provided to further ensure that the stent stays in place on the expandable portion of the delivery catheter 11, and to prevent abrasion of the body lumen by the open surface of the stent 10 during delivery to the desired arterial location. Other means for securing the stent 10 onto the balloon 14 may also be used, such as providing collars or ridges on the ends of the working portion, i.e., the cylindrical portion, of the balloon.

Each radially expandable cylindrical element 12 of the stent 10 can be independently expanded, so that the balloon 14 can also have an inflated shape that is not cylindrical, e.g., tapered, to facilitate implantation of the stent 10 in a variety of body lumen shapes.

In a preferred embodiment, the delivery of the stent 10 is accomplished in the following manner. The stent 10 is first mounted onto the inflatable balloon 14 on the distal extremity of the delivery catheter 11. The stent may be "crimped" down onto the balloon to ensure a low profile. The catheter-stent assembly can be introduced into the vasculature of the patient in a conventional Seldinger technique through a guiding catheter (not shown). A guidewire 18 is disposed across the damaged arterial section with the detached or dissected lining 16 and then the catheter-stent assembly is advanced over a guidewire 18 within the artery 15 until the stent 10 is directly under the detached lining 16. The balloon 14 of the catheter is expanded, expanding the stent 10 against the artery 15, which is illustrated in Fig. 2. While not shown in the drawing, the artery 15 is preferably expanded slightly by the expansion of the stent 10 to seat or otherwise fix the stent 10 to prevent movement. In some circumstances during the treatment of stenotic portions of an artery, the artery may have to be expanded considerably in order to facilitate passage of blood or other fluid therethrough.

The stent 10 serves to hold open the artery 15 after the catheter 11 is withdrawn, as illustrated by Fig. 3. Due to the formation of the stent 10 from an elongated tubular member, the undulating component of the cylindrical elements of the stent 10 is relatively flat in transverse cross-section, so that when the stent is expanded, the cylindrical elements are pressed into the wall of the artery 15, and as a result, do not interfere with the blood flow through the artery 15. The cylindrical elements 12 of stent 10 which are pressed into the wall of the artery 15 eventually will be covered with endothelial cell growth, which growth further minimizes blood flow interference. The serpentine pattern of the cylindrical sections 12 provide good tacking characteristics to prevent stent movement within the artery. Furthermore, the closely-spaced cylindrical elements 12 at regular intervals provide uniform support for the wall of the artery 15 and, consequently, are well adapted to tack up and hold in place small flaps or dissections in the wall of the artery 15 as illustrated in Figs. 2 and 3.

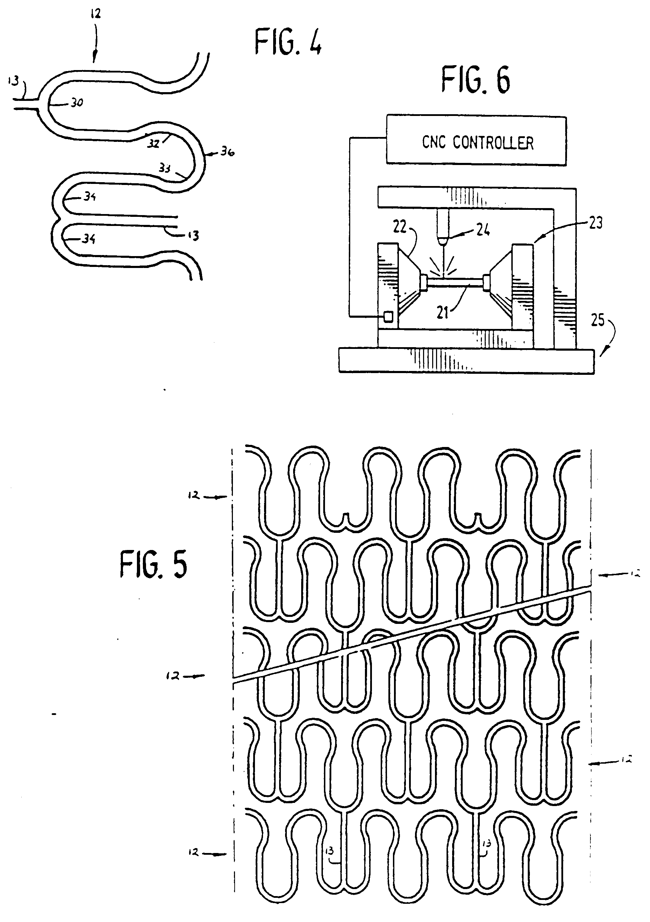

In the preferred embodiment, as depicted in Figs. 4 and 5, the stresses involved during expansion from a low profile to an expanded profile are much more evenly distributed among the various peaks and valleys. As seen in Fig. 4, a portion of cylindrical element 12 of stent 10 illustrates the irregular serpentine pattern having a plurality peaks and valleys which aids in the even distribution of expansion forces. Interconnecting members 13 serve to connect adjacent valleys of cylindrical element 12 as described above. During expansion, the portion 34 located in the region of the valley where interconnecting member 13 is connected, is the stiffest structure during deformation and the peak portion 36 the least stiff. Thus, a larger radius at portion 34 allows it to begin expanding sooner and at a more uniform rate as compared to the expansion of peak portion 36.

Due to this design, portion 34 is the stiffest structure and peak portion 36 is the least stiff structure, which accounts for the different stresses arising during expansion. Also, the least stiff structure, peak portion 36, is positioned between portion 34 and valley portion 30 which are stiffer structures. To even out the stresses, peak portion 36 has different curvatures at regions 32 and 33. Region 32 has a larger radius than region 33 and will expand more easily. Since region 33 is adjacent the stiffer area of portion 34, both region 33 and portion 34 will expand more uniformly, and will more evenly distribute the expansion stresses. Further, valley portion 30 and portion 34 also have a larger diameter to even out the expansion forces in relation to peak portion 36. Due to the novel structure as described, the shortcomings of the prior art, which include out-of-plane twisting of the metal, is avoided. These differing degrees of curvature along the peak portion 36 allow for the more even expansion of the cylindrical element 12 as a whole. Additionally, valley portion 34 can have differing degrees of curvature to compensate for the different stress levels during expansion. After expansion, portions of the various elements will turn outwardly, forming small projections which will embed in the vessel wall. For example, the tip of peak portion 36 tips outwardly upon expansion a sufficient amount to embed into the vessel wall and help secure the implanted stent. Upon expansion, the projecting peak 36 provides an outer wall surface on the stent that is not smooth, but instead has a plurality of projecting peaks 36 all along the outer wall surface. While the projections assist in securing the stent in the vessel wall, they are not sharp so as to cause trauma or damage to the vessel wall.

One important feature of the illustrated embodiment is the capability of the stent to expand from a low-profile diameter to a diameter much greater than heretofore was available, while still maintaining structural integrity in its expanded state. Due to its novel structure, the stent has an overall expansion ratio of 1.0 up to about 4.0 using certain compositions of stainless steel. For example, a 316L stainless steel stent can be radially expanded from a diameter of 1.0 unit up to a diameter of about 4.0 units, which deforms the structural members beyond their elastic limits. The stent still retains its structural integrity in the expanded state and it serves to hold open the vessel in which it is implanted. Materials other than 316L stainless steel may give higher or lower expansion ratios without sacrificing structural integrity.

The illustrated stent 10 can be made in many ways. One method of making the stent is to coat a thin-walled tubular member, such as a stainless steel hypotube, with a material which is resistive to chemical etchants, remove portions of the coating to expose underlying hypotubing which is to be removed, but leave coated portions of the hypotubing in the desired pattern for the stent so that subsequent etching will remove the exposed portions of the metallic tubing. The coated portion of the metallic tube is in the desired shape of the stent. An etching process avoids the necessity of removing burrs or slag inherent in conventional or laser machining processes. It is preferred to remove the etchant-resistive material by means of a machine-controlled laser as illustrated schematically in Fig. 6.

A coating is applied to a length of tubing which, when cured, is resistive to chemical etchants. A coating sold under the tradename "Blue Photoresist" by the Shipley Company in San Jose, California, is an example of suitable commercially available photolithographic coatings. The coating preferably is applied by electrophoretic deposition.

To ensure that the surface finish is reasonably uniform, one of the electrodes used for the electrochemical polishing is a doughnut-shaped electrode which is placed about the central portion of the tubular member.

The tubing may be made of suitable biocompatible material such as stainless steel, titanium, tantalum, super-elastic nickel-titanium (NiTi) alloys and even high strength thermoplastic polymers. The stent diameter is very small, so the tubing from which it is made must necessarily also have a small diameter. For devices that are intended to be used in the vessels of the coronary vasculature, for example, following performance of percutaneous transluminal coronary angioplasty (PTCA) applications, the stent typically has an outer diameter on the order of about 1.65 mm (0.065 inch) in the unexpanded condition, and can be expanded to have an outer diameter of 5.08 mm (0.2 inch) or more. The wall thickness of the hypotubing is typically about 0.08 mm (0.003 inch). For stents implanted in other body lumens, such as in the course of percutaneous transluminal angioplasty (PTA) procedures performed in non-coronary vessels applications, the dimensions of the hypotubing correspondingly are larger.

In the instance when the stent is made from plastic, it may have to be heated within the arterial site where the stent is to be expanded, to facilitate the deformation of the stent. Once expanded, it would then be cooled to retain its expanded state. The stent conveniently may be heated by heating the fluid within the balloon or the balloon directly by a known method. The stent may also be made of materials such as super-elastic NiTi alloys. In this case the stent would be formed full size but deformed (e.g., compressed) into a smaller diameter onto the balloon of the delivery catheter to facilitate transfer to a desired intraluminal site. The stress induced by the deformation transforms the stent from a martensite phase to an austenite phase and, upon release of the force, when the stent reaches the desired intraluminal location, allows the stent to expand due to the transformation back to the martensite phase.

Referring to Fig. 6, the coated tubing 21 is put in a rotatable collet fixture 22 of a machine-controlled apparatus 23 for positioning the tubing 21 relative to a laser 24.

According to machine-encoded instructions, the tubing 21 is rotated and moved longitudinally relative to the laser 24 which also is machine-controlled. The laser selectively removes the etchant-resistive coating on the tubing by ablation, and a pattern is formed such that the surface of the tube that is to be removed by a subsequent chemical etching process is exposed. The surface of the tube therefore is left coated in the discrete pattern of the finished stent.

One system for removing the coating on the tubing includes the use of an 80 watt $CO_2$ laser, such as that sold under the tradename "Coherent Model 44," in pulse mode (0.3 ms pulse length); 48 ma key current and 48 W key power with 0.75 W average power; at 100 Hz; Anorad FR=20; 0.017 bars (12.5 Torr); with no assist gas. Another system for removing the coating on the tubing includes the use of the same laser only operating in the rapid switching (Q-switched) mode, generating about 1.6 kHz for approximately 500 ns intervals (i.e., mechanically chopping the beam). Low pressure air is directed through the fine focus head to ensure that no vapor contacts the lens. The assist gas jet assembly on the laser unit may be removed to allow a closer proximity of the fine focus head and the collet fixture. Optimum focus is set at the surface of the tubing. Cured photoresist coating readily absorbs the energy of the $CO_2$ wavelength, so that it can be readily removed by the laser. The process of photoresist ablation with the laser still leaves a very thin film of photo-resist which must be removed. Known methods of removal such as plasma etching, can be used to remove the photo-resist film. For manufacturing efficiency a coated 10,16 cm (4.0 inch) length of 1.52 mm (0.06 inch) stainless steel tubing is preferred, and four stents can be patterned on that length of tubing. Three tabs or webs between stents provide good handling characteristics for the tubing after the etching process. It is also possible to use a neodymium-doped ytrium aluminum garnet (Nd:YAG) laser, or an excimer or ion laser in place of a $CO_2$ laser. The settings and power requirements of these other lasers would be different than that described for the $CO_2$.

The process of patterning the resistive coating on the stent is automated, except for loading and unloading the length of tubing. Referring again to Fig. 6 it may be done, for example, using a computer-numerical-control (CNC)-opposing collet fixture 22 for axial rotation of the length of tubing, in conjunction with a CNC X/Y table 25 to move the length of tubing axially relative to a machine controlled laser as described. The entire space between collets can be patterned using the $CO_2$ laser set-up of the foregoing example. The program for control of the apparatus is dependent on the particular configuration used and the pattern to be ablated in the coating, but is otherwise conventional.

This process makes possible the application of present photo-lithography technology in manufacturing the stents. While there is presently no practical way to mask and expose a tubular photoresist coated part of the small size required for making intravascular stents, the foregoing steps eliminate the need for conventional masking techniques.

After the coating is selectively thus ablated, the tubing is removed from the collet fixture 22. Next, wax such as that sold under the tradename "ThermoCote N-4," is heated to preferably just above its melting point, and inserted into the tubing under vacuum or pressure. After the wax has solidified upon cooling, it is reheated below its melting point to allow softening, and a smaller diameter stainless steel shaft is inserted into the softened wax to provide support. The tubing then is etched chemically in a conventional manner. After cutting the tabs connecting the stents any surface roughness or debris from the tabs is removed. The stents preferably are polished electrochemically in an acidic aqueous solution, such as a solution sold under the tradename ELECTRO-GLO #300, by the ELECTRO-GLO CO., INC. in Chicago, Illinois, U.S.A., which is a mixture of sulfuric acid, carboxylic acids, phosphates, corrosion inhibitors and a biodegradable surface active agent. The bath temperature is maintained at about 37.8 - 57.2°C (110-135°F), and the current density is about 0.4 to about 1.5 amps per 6,45 cm$^2$ (in$^2$). Cathode-to-anode area ratio should be at least about two to one.

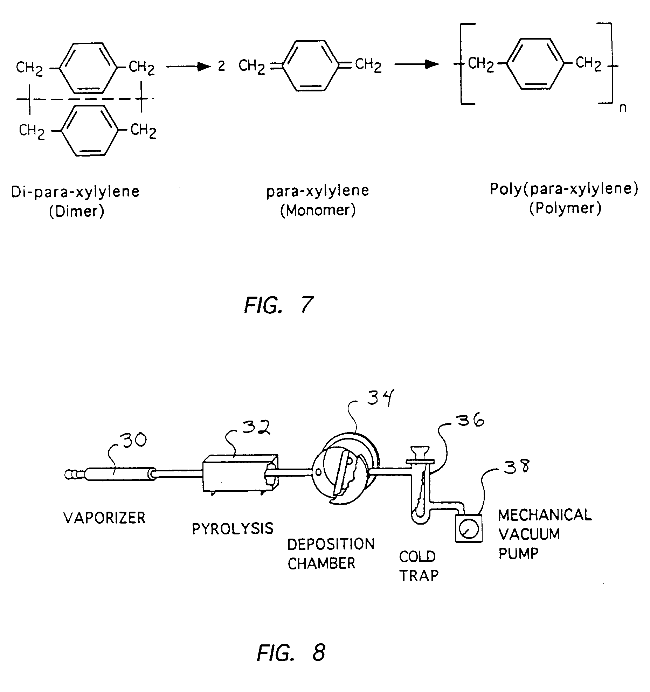

The stents are treated further by applying a coating of parylene over the stent by vacuum vapor deposition, as is illustrated by Figs. 7 and 8. The parylene polymer coating typically is deposited from the vapor phase by a process which is similar to vacuum metalization. The presently preferred form of parylene is Parylene N, poly-(para-xylylene). Also suitable is Parylene C, poly-(chloro-para-xylylene), which differs from the parent compound Parylene N, poly-(para-xylylene), in that one chlorine is substituted for one hydrogen in each aromatic ring in Parylene C. Parylene D, poly (dichloro-para-xylylene), is another member of the parylene family, and is produced from the same Parylene N monomer by the substitution of chlorine for two of the aromatic hydrogens. Parylene D has properties similar to those of Parylene C, with the additional ability to withstand higher temperatures. In certain applications, Parylene D also may be a suitable coating for a stent.

With reference to Figs. 7 and 8, illustrating the deposition of a parylene coating on the stent, the vacuum deposition process involves first sublimating the dimer in a vacuum at a temperature of from approximately 100 to 300°C (212-572°F), and preferably at approximately 150°C (302°F), in a vaporizer 30. The sublimed vapors of the dimer are then subjected to pyrolisys in a pyrolisys chamber 32 at a temperature of from about 500 to about 750°C (932-1382°F), and preferably at about 680°C (1256°F) to yield a stable monomeric diradical in a vacuum of approximately .67 mbars (0.5 Torr). The monomer then enters a room temperature deposition chamber 34 in which the stent has been placed, at approximately 25°C (77°F), and at about .13 mbars (0.1 Torr), where the monomer vapors contact and condense on the stent, to form the polymeric parylene coating on the surface of the stent. Vapors which have not condensed in the deposition chamber are removed in a cold trap 36, at a reduced temperature of -70°C (-158°F), for example, which protects the mechanical vacuum pump 38 from contamination. The stent is coated with a thin, uniform layer of parylene with a thickness of from about 2.5 μm to about 5 μm (0.0001 inch to about 0.0002 inch), to provide maximal adhesion of the parylene coating to the surface of the stent. The thickness of the parylene coating in this range is critical to substantially avoid altering the profile and mechanical characteristics of the stent. It should be noted that the stents can be made of various other metals other than stainless steel, and can be coated with parylene, to protect the body from thrombogenesis and neointimal hyperplasia.

Example

In comparative studies, seven millimeter long stainless steel stents coated with poly(para-xylylene) were implanted in the femoral arteries of rabbits treated with aspirin one day prior to stent placement. The stents were placed in the iliac artery with a 3 millimeter percutaneous transluminal coronary angioplasty (PTCA) balloon catheter, which was introduced through a surgical arteriotomy. All animals also received an intravenous dose of heparin at the time of the arteriotomy. After fourteen days, the arteries at the site of the stents were examined for thrombosis, and for smooth muscle cell proliferation as an indication of neointimal hyperplasia. While conventional uncoated intravascular stents are typically subject to an incidence of up to 42% of acute and subacute thrombosis, and high rates of neointimal hyperplasia, for stents embodying the invention and coated with a thickness of 3 μm (3 microns) of parylene, the percentage of stents that were completely thrombosed were found to have been reduced to zero.

An ultimate measure of effectiveness of stent placement is luminal patency. The degree of thrombosis can be combined with the extent of intimal hyperplasia, and averaged for a sample of stent placements to provide an overall index of stent performance, according to the following formula:

$$\text{Stenosis Index} = \frac{(n * 100) + (m * \% \text{ occlusion})}{m + n}$$

where n is the number of completely thrombosed stents, and m is the number of patent, or open, stents. Completely thrombosed stents receive a score of 100. Partial luminal obstruction from smooth muscle proliferation and thrombosis of the vessel wall were measured with computer-aided planimetry, and received a score of from 0, indicating no stenosis, to 99, indicating nearly complete occlusion.

It was observed that while the parylene coating did not significantly worsen or reduce neointimal hyperplasia, addition of parylene coating reduced stenosis by 34% according to this index of overall stent performance in terms of prevention of stenosis, two weeks after stent placement. Overall stent patency and reduction of thrombogenesis were therefore found to be markedly enhanced by coating the stent with parylene. Coating slotted-tube stents with parylene also reduced incidence of stent occlusion due to thrombosis from 42% to 8%. It has been speculated that the parylene coating may serve to change the electropotential of the surface of the stent, and that there may be a correspondence between the difference in electropotential of the blood and the stent with thrombosis and restenosis.

In view of the foregoing, it has been demonstrated that the described embodiment provides for an intravascular stent with a coating which reduces thrombogenesis and restenosis. While previous uses of parylene as a coating have been for purposes of protecting devices such as electrodes and circuits from corrosion, degradation and other damage in the body, the parylene coating on the illustrated stent serves to protect the body from injury by the stent, as is evidenced by the reduction of thrombogenesis and restenosis. In view of the reduction of thrombogenesis, anticoagulant regimes can be reduced, in order to reduce bleeding and other vascular complications. The use of parylene as an antithrombogenic coating on a metal stent embodying the invention can reduce thrombosis, neointimal hyperplasia and other adverse physiological reactions contributing to restenosis.

While the invention has been illustrated and described here in terms of its use as an intravascular stent, it will be apparent to those skilled in the art that the stent can be used in other instances in other body lumens. Since the illustrated stent has the feature of being expandable to very large diameters, while retaining its structural integrity, it is particularly well suited for implantation in almost any vessel where such devices are used. This feature, coupled with limited longitudinal contraction of the stent when it is radially expanded, provides a highly desirable support member for all vessels in the body. The ability of the stent to reduce thrombosis and restenosis also make the stent highly desirable for use in all vessels in the body. Other modifications and improvements may be made without departing from the scope of the invention.

**Claims**

1.  A longitudinally flexible stent (10) for implanting in a body lumen and expandable from a contracted condition to an expanded condition, comprising:

    a plurality of adjacent cylindrical elements (12) which are independently expandable in a radial direction and arranged in alignment along a longitudinal stent axis, said cylindrical elements (12) being formed in a serpentine wave pattern transverse to the longitudinal axis and containing a plurality of alternating peaks (36) and valleys (34); and
    at least one interconnecting member (13) extending between adjacent cylindrical elements (12) and connecting them to one another, characterised in that said serpentine wave pattern is irregular and said stent (10) further comprises an anti-thrombogenic coating covering said plurality of cylindrical elements (12) and said at least one interconnecting member (13), said coating comprising a parylene polymer selected from the group consisting of poly-(chloro-para-xylylene), poly-(dichloro-para-xylylene), and poly (para-xylylene).

2.  The stent (10) of claim 1, wherein said irregular serpentine pattern (12,34,36) contains varying degrees of curvature in regions of said peaks (36) and valleys (34) which are adapted so that the radial expansion of said adjacent cylindrical elements (12) is generally uniform around their circumferences during the expansion of the stent (10) from its contracted condition to its expanded condition.

3.  The stent (10) of claim 1 or claim 2, wherein the degree of curvature along said peaks (36) and valleys (34) is determined relative to the anticipated level of stress created during the expansion of the stent (10) from its contracted condition to its expanded condition.

4.  The stent (10) of any preceding claim, wherein the degrees of curvature along adjacent peaks (36) and valleys (34) of said irregular serpentine pattern (12,34,36) are different.

5.  The stent (10) of any preceding claim, wherein the degree of curvature along said peaks (36) of said irregular serpentine pattern (12,34,36) is different in immediately adjacent regions.

6.  The stent (10) of any preceding claim, wherein the degree of curvature along said peaks (36) and valleys (34) is chosen so that the bending stress developed at said peaks (36) and valleys (34) during the radial expansion of said cylindrical elements (12) is uniform around the circumference of said cylindrical elements (12).

7.  The stent (10) of any preceding claim, wherein said at least one interconnecting member (13) connects a valley (34) of one cylindrical element (12) with a valley (34) of an adjacent cylindrical element (12).

8.  The stent (10) of any preceding claim, wherein said interconnecting member (13) is unitary with said valley (34) of one cylindrical element (12) and said valley (34) of said adjacent cylindrical element (12).

9.  The stent (10) of any preceding claim, wherein said adjacent cylindrical elements (12) are connected to each other by a plurality of said interconnecting members (13) whereby said stent (10) may expand from a first smaller diameter to a second enlarged diameter without an appreciable change in overall length.

10. The stent (10) of any preceding claim, wherein said cylindrical elements (12) cooperate to define a generally cylindrical surface and wherein said peaks (36) project outwardly from said surface upon expansion.

11. The stent (10) of any preceding claim, wherein said stent (10) is formed of a biocompatible material selected from the group consisting of stainless steel, tungsten, tantalum, super-elastic nickel-titanium (NiTi) alloys, and thermoplastic polymers.

12. The stent (10) of any preceding claim, wherein said stent (10) has a radial expansion ratio of about 1 up to about 4.0.

13. The stent (10) of any preceding claim, wherein the stent (10) is formed from a single piece of tubing (21).

14. A kit of parts for delivering an intraluminal stent in a body lumen comprising:

an elongated stent delivery catheter (11) having a proximal end and a distal end, and an expandable member (14) adjacent the distal end;
a longitudinally flexible stent (10) expandable from a contracted condition to an expanded condition, comprising:

a plurality of adjacent cylindrical elements (12) which are independently expandable in a radial direction and arranged in alignment along a longitudinal stent axis, said cylindrical elements (12) being formed in a serpentine wave pattern transverse to the longitudinal axis and containing a plurality of alternating peaks (36) and valleys (34); and
at least one interconnecting member (13) extending between adjacent cylindrical elements (12) and connecting them to one another; and means for expanding said expandable member (14) to, in turn, radially expand said stent (10) and implant said stent in said body lumen, characterised in that said serpentine wave pattern is irregular and said stent (10) further comprises an anti-thrombogenic coating covering said plurality of cylindrical elements (12) and said at least one interconnecting member (13), said coating comprising a parylene polymer selected from the group consisting of poly-(chloro-para-xylylene), poly-(dichloro-para-xylylene, and poly(para-xylylene).

15. The kit of parts of claim 14, wherein said stent (10) is in accordance with any of claims 2 to 13.

**Patentansprüche**

1. Längsflexibler Stent (10) zum Implantieren in ein Körperlumen, der von einem kontrahierten Zustand zu einem aufgeweiteten Zustand aufweitbar ist, umfassend:

eine Mehrzahl benachbarter zylindrischer Elemente (12), die in radialer Richtung unabhängig aufweitbar sind und entlang einer Stentlängsachse ausgerichtet angeordnet sind, wobei die zylindrischen Elemente (12) quer zur Längsachse in einem Schlangenwellenmuster ausgebildet sind und eine Mehrzahl abwechselnder Gipfel (36) und Täler (34) enthalten; und
zumindest ein Verbindungselement (13), welches sich zwischen benachbarten zylindrischen Elementen (12) erstreckt und diese miteinander verbindet,
dadurch gekennzeichnet, daß
das Schlangenwellenmuster unregelmäßig ist und der Stent (10) ferner eine antithrombogene Beschichtung aufweist, welche die Mehrzahl zylindrischer Elemente (12) und das zumindest eine Verbindungselement (13) bedeckt, wobei die Beschichtung ein Parylenpolymer aufweist, das aus der Gruppe gewählt ist, die aus Poly(chlor-para-xylylen), Poly(dichlor-para-xylylen) und Poly(para-xylylen) besteht.

2. Stent (10) nach Anspruch 1, wobei das unregelmäßige Schlangenmuster (12, 34, 36) in Bereichen der Gipfel (36) und Täler (34) unterschiedliche Krümmungsgrade aufweist, die derart ausgelegt sind, daß die radiale Aufweitung der benachbarten zylindrischen Elemente (12) um ihre Umfänge herum während des Aufweitens des Stents (10) von seinem kontrahierten Zustand zu seinem aufgeweiteten Zustand allgemein gleichmäßig ist.

3. Stent (10) nach Anspruch 1 oder Anspruch 2, wobei der Krümmungsgrad entlang den Gipfeln (36) und Tälern (34) in Bezug auf den zu erwartenden Belastungsgrad bestimmt ist, der während des Aufweitens des Stents (10) von seinem kontrahierten Zustand zu seinem aufgeweiteten Zustand erzeugt wird.

4. Stent (10) nach einem der vorhergehenden Ansprüche, wobei die Krümmungsgrade entlang benachbarter Gipfel (36) und Täler (34) des unregelmäßigen Schlangenmusters (12, 34, 36) unterschiedlich sind.

5. Stent (10) nach einem der vorhergehenden Ansprüche, wobei der Krümmungsgrad entlang der Gipfel (36) des unregelmäßigen Schlangenmusters (12, 34, 36) in unmittelbar benachbarten Bereichen unterschiedlich ist.

6. Stent (10) nach einem der vorhergehenden Ansprüche, wobei der Krümmungsgrad entlang der Gipfel (36) und Täler (34) derart gewählt ist, daß die Biegebelastung, die während der radialen Aufweitung der zylindrischen Elemente (12) an den Gipfeln (36) und Tälern (34) entsteht, um den Umfang der zylindrischen Elemente (12) herum gleichmäßig ist.

7. Stent (10) nach einem der vorhergehenden Ansprüche, wobei das zumindest eine Verbindungselement (13) ein Tal (34) eines zylindrischen Elements (12) mit einem Tal (34) eines benachbarten zylindrischen Elements (12) verbindet.

8. Stent (10) nach einem der vorhergehenden Ansprüche, wobei das Verbindungselement (13) mit dem Tal (34) eines zylindrischen Elements (12) und dem Tal (34) des benachbarten zylindrischen Elements (12) einheitlich ist.

9. Stent (10) nach einem der vorhergehenden Ansprüche, wobei die benachbarten zylindrischen Elemente (12) miteinander durch eine Mehrzahl von Verbindungselementen (13) verbunden sind, wodurch der Stent (10) ohne merkliche Änderung seiner Gesamtlänge von einem ersten kleineren Durchmesser zu einem zweiten vergrößerten Durchmesser aufgeweitet werden kann.

10. Stent (10) nach einem der vorhergehenden Ansprüche, wobei die zylindrischen Elemente (12) zur Bildung einer allgemein zylindrischen Oberfläche zusammenwirken und wobei die Gipfel (36) beim Aufweiten von der Oberfläche nach außen vorstehen.

11. Stent (10) nach einem der vorhergehenden Ansprüche, wobei der Stent (10) aus einem biokompatiblen Material gebildet ist, das aus der Gruppe gewählt ist, die aus rostfreiem Stahl, Wolfram, Tantal, superelastischen Nickel-Titan (NiTi) Legierungen und thermoplastischen Polymeren besteht.

12. Stent (10) nach einem der vorhergehenden Ansprüche, wobei der Stent (10) ein radiales Aufweitungsverhältnis von etwa 1 bis zu etwa 4,0 aufweist.

13. Stent (10) nach einem der vorhergehenden Ansprüche, wobei der Stent (10) aus einem einzigen Rohrstück (21) gebildet ist.

14. Teilesatz zur Ausgabe eines intraluminalen Stents in ein Körperlumen, umfassend:

einen langgestreckten Stentausgabekatheter (11) mit einem proximalen Ende und einem distalen Ende und einem aufweitbaren Element (14) nahe dem distalen Ende;
einen längsflexiblen Stent (10), der von einem kontrahierten Zustand zu einem aufgeweiteten Zustand aufweitbar ist, umfassend:
eine Mehrzahl benachbarter zylindrischer Elemente (12), die in radialer Richtung unabhängig aufweitbar sind und längs einer Stentlängsachse ausgerichtet angeordnet sind, wobei die zylindrischen Elemente (12) quer zur Längsachse in einem Schlangenwellenmuster ausgebildet sind und eine Mehrzahl abwechselnder Gipfel (36) und Täler (34) enthalten; und
zumindest ein Verbindungselement (13), welches sich zwischen benachbarten zylindrischen Elementen (12) erstreckt und diese miteinander verbindet; und Mittel zum Aufweiten des aufweitbaren Elements (14), um wiederum den Stent (10) radial aufzuweiten und den Stent in das Körperlumen zu implantieren, dadurch gekennzeichnet, daß das Schlangenwellenmuster unregelmäßig ist und der Stent (10) ferner eine antithrombogene Beschichtung aufweist, welche die Mehrzahl zylindrischer Elemente (12) und das zumindest eine Verbindungselement (13) bedeckt, wobei die Beschichtung ein Parylenpolymer aufweist, das aus der Gruppe gewählt ist, die aus Poly(chlor-para-xylylen), Poly(dichlor-para-xylylen) und Poly(para-xylylen) besteht.

15. Teilesatz nach Anspruch 14, wobei der Stent (10) nach einem der Ansprüche 2 bis 13 ist.

**Revendications**

1. Stent (10) flexible dans le sens longitudinal destiné à être implanté dans une lumière de l'organisme et expansible entre un état comprimé et un état expansé, comprenant :

une pluralité d'éléments cylindriques (12) adjacents qui sont indépendamment expansibles dans une direction radiale et disposés en alignement le long d'un axe longitudinal du stent, les dits éléments cylindriques (12) étant façonnés en un motif ondulé en serpentin transversal par rapport à l'axe longitudinal et contenant une pluralité de pics (36) et de vallées (34) alternés ; et

au moins un membre d'interconnexion (13) s'étendant entre les éléments cylindriques adjacents (12) et les reliant les uns aux autres, caractérisé en ce que ledit motif ondulé en serpentin est irrégulier et en ce que ledit stent (10) comprend en outre un revêtement anti-thrombogène couvrant ladite pluralité d'éléments cylindriques (12) et ledit au moins un membre d'interconnexion (13), ledit revêtement comprenant un polymère parylène choisi dans le groupe consistant en poly-(chloro-para-xylylène), poly-(dichloro-para-xylylène), et poly-(para-xylylène).

2. Stent (10) selon la revendication 1, dans lequel ledit motif en serpentin irrégulier (12, 34, 36) contient des degrés variables de courbure dans des régions desdits pics (36) et desdites vallées (34) qui sont adaptés de manière que l'expansion radiale desdits éléments cylindriques adjacents (12) soit généralement uniforme sur leurs circonférences durant l'expansion du stent (10) entre son état comprimé et son état expansé.

3. Stent (10) selon la revendication 1 ou la revendication 2, dans lequel le degré de courbure le long desdits pics (36) et desdites vallées (34) est déterminé en fonction du niveau de contrainte anticipé créé durant l'expansion du stent (10) entre son état comprimé et son état expansé.

4. Stent (10) selon l'une quelconque des revendications précédentes, dans lequel les degrés de courbure le long des pics (36) et des vallées (34) adjacents dudit motif en serpentin irrégulier (12, 34, 36) sont différents.

5. Stent selon l'une quelconque des revendications précédentes, dans lequel le degré de courbure le long desdits pics (36) dudit motif en serpentin irrégulier (12, 34, 36) est différent de celui des régions immédiatement adjacentes.

6. Stent (10) selon l'une quelconque des revendications précédentes, dans lequel le degré de courbure le long desdits pics (36) et desdites vallées (34) est choisi de manière que la contrainte de flexion développée au niveau desdits pics (36) et desdites vallées (34) durant l'expansion radiale desdits éléments cylindriques (12) soit uniforme sur la circonférence desdits éléments cylindriques (12).

7. Stent (10) selon l'une quelconque des revendications précédentes, dans lequel ledit au moins un membre d'interconnexion (13) relie une vallée (34) d'un élément cylindrique (12) à une vallée (34) d'un élément cylindrique (12) adjacent.

8. Stent (10) selon l'une quelconque des revendications précédentes, dans lequel ledit membre d'interconnexion (13) est solidaire de ladite vallée (34) d'un élément cylindrique (12) et de ladite vallée (34) dudit élément cylindrique (12) adjacent.

9. Stent (10) selon l'une quelconque des revendications précédentes, dans lequel lesdits éléments cylindriques (12) adjacents sont reliés les uns aux autres par une pluralité desdits membres d'interconnexion (13) grâce auxquels ledit stent (10) peut se dilater entre un premier petit diamètre et un second diamètre élargi sans modification appréciable de sa longueur globale.

10. Stent (10) selon l'une quelconque des revendications précédentes, dans lequel lesdits éléments cylindriques (12) coopèrent pour définir une surface généralement cylindrique et dans lequel lesdits pics (36) font saillie vers l'extérieur depuis ladite surface lors de l'expansion.

11. Stent (10) selon l'une quelconque des revendications précédentes, dans lequel ledit stent (10) est formé d'un matériau biocompatible choisi dans le groupe consistant en acier inoxydable, tungstène, tantale, alliages super-élastiques de nickel et de titane (NiTi), et polymères thermoplastiques.

12. Stent (10) selon l'une quelconque des revendications précédentes, dans lequel ledit stent (10) possède un coefficient d'expansion radiale d'environ 1 à environ 4,0.

13. Stent (10) selon l'une quelconque des revendications précédentes, dans lequel ledit stent (10) est constitué d'une tubulure (21) en une seule pièce.

14. Trousse de pièces destinée à délivrer un stent intraluminal dans une lumière de l'organisme comprenant :

un cathéter de mise en place de stent allongé (11) comportant une extrémité proximale et une extrémité distale, et un membre expansible (14) adjacent à l'extrémité distale ;
un stent (10) flexible dans le sens longitudinal expansible entre un état comprimé et un étant expansé, comprenant :
une pluralité d'éléments cylindriques (12) adjacents qui sont indépendamment expansibles dans une direction radiale et disposés en alignement le long d'un axe longitudinal du stent, lesdits éléments cylindriques (12) étant façonnés en un motif ondulé en serpentin transversal par rapport à l'axe longitudinal et contenant une pluralité de pics (36) et de vallées (34) alternés ; et

au moins un membre d'interconnexion (13) s'étendant entre les éléments cylindriques (12) adjacents et les reliant les uns aux autres ; et un moyen pour dilater ledit membre expansible (14), afin, ensuite, de dilater ledit stent (10) dans le sens radial et d'implanter ledit stent dans ladite lumière de l'organisme, caractérisée en ce que ledit motif ondulé en serpentin est irrégulier et en ce que ledit stent (10) comprend en outre un revêtement anti-thrombogène couvrant ladite pluralité d'éléments cylindriques (12) et ledit au moins un membre d'interconnexion (13), ledit revêtement comprenant un polymère parylène choisi dans le groupe consistant en poly-(chloro-para-xylylène), poly-(dichloro-para-xylylène), et poly-(para-xylylène).

15. Trousse de pièces selon la revendication 14, dans laquelle ledit stent (10) est tel que défini dans l'une quelconque des revendications 2 à 13.

FIG.1

FIG.2

FIG.3

EP 0 679 373 B1

FIG. 4

FIG. 6

FIG. 5

13

Di-para-xylylene (Dimer) → para-xylylene (Monomer) → Poly(para-xylylene) (Polymer)

*FIG. 7*

*FIG. 8*